# EUROPÄISCHE PATENTSCHRIFT

(11) **EP 2 421 492 B1**
(45) Veröffentlichungstag und Bekanntmachung des Hinweises auf die Patenterteilung: **16.01.2013**
(21) Anmeldenummer: 10717541.6
(22) Anmeldetag: 20.04.2010
(51) Int. Cl.: A61J 1/14, A61J 1/20

(54) **FÜR DIE ENTNAHME VON MEDIZINISCHEN FLÜSSIGKEITEN AUS VERPACKUNGEN GEEIGNETE VORRICHTUNG UND DAMIT AUSGESTATTETE VERPACKUNG**
DEVICE SUITABLE FOR REMOVING MEDICAL FLUIDS FROM PACKAGINGS AND PACKAGING PROVIDED HEREWITH
DISPOSITIF APPROPRIÉ POUR LE PRÉLÈVEMENT DE FLUIDES MÉDICAUX CONTENUS DANS DES EMBALLAGES ET EMBALLAGE ÉQUIPÉ DE CE DERNIER

(30) Priorität: 23.04.2009 DE 102009019503; 20.07.2009 DE 102009033908
(43) Veröffentlichungstag der Anmeldung: 29.02.2012
(73) Patentinhaber: Van Der Wal, Wiebe, 63697 Hirzenhain (DE); Zaubitzer, Thomas, 66484 Klainsteinhausen (DE)
(72) Erfinder: VAN DER WAL, Wiebe, 63697 Hirzenhain (DE)
(74) Vertreter: Dreiss
(86) Internationale Anmeldenummer: PCT/EP2010/002393
(87) Internationale Veröffentlichungsnummer: WO 2010/121772

(56) Entgegenhaltungen:
- WO-A1-93/02724
- WO-A2-2006/138184
- DE-A1-102005 015 504
- FR-A1- 2 851 912

## Beschreibung

Die vorliegende Erfindung bezieht sich auf eine für die Entnahme von insbesondere medizinischen Flüssigkeiten aus vorzugsweise Einwegverpackungen, wie Infusions- oder Transfusionsbeutel oder -flaschen geeignete Vorrichtung, nach dem Oberbegriff des Anspruchs 1.

Derartige Vorrichtungen in Form von Verbindungsstücken werden am im Gebrauch unteren Ende von Infusions- oder Transfusionsbeuteln mit ihrer verpackungsseitigen Öffnung direkt oder mittels einer kurzen flexiblen Leitung eingeschweißt oder eingeklebt, wobei sie eine Verschlussmembran aufweisen, die von einem Originalitätsverschluss abgedeckt ist. Zum Gebrauch des Infusions- oder Transfusionsbeutels wird der Originalitätsverschluss entfernt oder abgetrennt und die Membran von einem Spike bzw. Einstechdorn oder einer Nadel oder dergleichen durchstochen, wobei der Einstechdorn oder dergleichen bspw. über eine Tropfkammer mit einer Infusions - oder Transfusionsleitung, die zum Patienten führt, verbunden ist. Entsprechendes gilt, wenn die Vorrichtung als Verschluss für Infusions- oder Transfusionsflaschen ausgebildet ist, (WO 93/02724 A1).

Bei den bekannten Vorrichtungen ist es möglich, am Ende einer Infusion oder Transfusion, bei der der betreffende Infusions - oder Transfusionsbeutel oder die Flasche nicht vollständig geleert worden ist, nach dem Herausziehen des betreffenden Einstechdorns, mit dem Verbindungsstück des Infusions- oder Transfusionsbeutels bzw. dem Verschluss einer Flasche erneut einen Einstechdorn mit einem Überleitungssystem zu einem anderen Patienten zu verwenden. Dies ist insbesondere dort möglich, wo selbstschließende Membrane verwendet werden. Diese Mehrfachnutzung eines Infusions- oder Transfusionsbeutels oder -flasche ist medizinisch bedenklich.

Aufgabe der vorliegenden Erfindung ist es deshalb eine Vorrichtung der eingangs genannten Art zu schaffen, die ein zerstörungsfreies Herausnehmen eines bereits eingebrachten und mit einem Überleitungssystem verbundenen und benutzten Einstechdorns verhindert bzw. unmöglich macht.

Zur Lösung dieser Aufgabe sind bei einer Vorrichtung der genannten Art die im Anspruch 1 angegebenen Merkmale vorgesehen.

Durch die erfindungsgemäßen Maßnahmen ist erreicht, dass ein einmal in das Verbindungsstück bzw. den Verschluss eingebrachter Einstechdorn oder dergleichen nach dem Durchstechen der Membran in dem Sicherungsring axial festgehalten und nicht mehr zurückgezogen werden kann, weil der Sicherungsring eine reibschlüssige bis formschlüssige umfangsseitige Aufnahme des Einstechdorns vornimmt. Außerdem ist keine zusätzliche Halterung des Einstechdorns im Verbindungsstück bzw. im Verschluss während der Infusion bzw. Transfusion notwendig.

Mit Hilfe einer Flachmembran gemäß den Merkmalen des Anspruchs 2 ist eine kostengünstige Lösung erreicht, da derartige Flachmembrane aus Bahnen entsprechenden Materials in schneller und einfacher Weise ausgestanzt werden können. Da ein die Flachmembran durchdringender Einstechdorn im axialen Sicherungs- bzw. Verriegelungselement axial gehalten ist, reicht auch die entsprechend geringere Dicke der Flachmembran aus.

Vorteilhafte Ausgestaltungen des Sicherungs- bzw. Verriegelungselementes ergeben sich aus den Merkmalen eines oder mehrerer der Ansprüche 3 bis 7. Insbesondere dadurch, dass das Sicherungs- bzw. Verriegelungselement aus nicht rostendem Stahl, vorzugsweise Federstahl medizinischer Qualität ist und mit scharfen Kanten versehen ist und der Einstechdorn üblicherweise aus einem Kunststoff ist, wird eine formschlüssige Verbindung durch radiales Eindringen der Zähne des axialen Sicherungs- bzw. Verriegelungselementes in die Umfangswandung des Einstechdorns erreicht. Dieses Element ist vorteilhaft als geschlossene oder offene Ringscheibe ausgebildet, welche letztere den Vorteil hat, dass aufgrund einer möglichen radialen Spreizung Einstechdorne unterschiedlicher Durchmesser aufgenommen werden können. Mit einem versuchten Herausziehen des Einstechdorn aus dem Verbindungsstück werden dessen Zähne weiter eingegraben, so dass ein zerstörungsfreies Herausziehen des Einstechdorns aus dem Verbindungsstück unmöglich gemacht ist. Außerdem wird beim Einstechen ein Verdrehen des Einstechdorns verhindert, so dass kein Materialabrieb an der Membran entstehen kann.

Eine vorteilhafte Anordnung und Fixierung des Sicherungsringes im Verbindungsstück bzw. Verschluss ergibt sich aus den Merkmalen nach Anspruch 8. Dabei kann es zweckmäßig sein, die Merkmale nach Anspruch 9 vorzusehen.

Mit den Merkmalen des Anspruchs 10 und ggf. denen des Anspruchs 11 ist ein weiterer wesentlicher Sicherheitsaspekt erreicht, nämlich der, dass jeder Gebrauch zusätzlich sichtbar wird.

Die vorliegende Erfindung bezieht sich nach Anspruch 12 und ggf. nach Anspruch 13 und/oder 14 sowie nach Anspruch 15 des weiteren auf eine Verpackung für insbesondere medizinische Flüssigkeiten, entweder in Form eines Infusions- oder Transfusionsbeutels oder in Form einer Infusions- oder Transfusionsflasche, der bzw. die mit einer Vorrichtung nach Anspruch 1 und/oder einem der folgenden versehen ist.

Weitere Vorteile der Selbstsicherungsstechverbindung sind: Der Verriegelungs- bzw. Sicherungsring verhindert das Drehen des Einstiegdorns; das Drehen verursacht Abrieb, welcher in die Infusionslösung geraten kann.

Die Aluminiums-Sicherheitsfolie, welche über der Einstichmembran angebracht ist, zeigt jede Manipulation auch nach dem Abziehen der Sterilitätskappe.
Der Sicherungs-Ring garantiert eine sichere und schnelle Stechverbindung unter allen Umständen.
Sichere Stechverbindung während Infusion unter allen Transportbedingungen.
Kein Auswechseln der Stechverbindung möglich.
Kein unvorhersehbares Lösen der Stechverbindungen durch Verhaken oder Verheddern der Infusionsleitung.
100% nur einmal brauchbare Stechverbindung.
Ideale und sichere sowie stabile Infusionsverbindung unter erschwerten Rettungsaktionen und für die Veterinärmedizin. Keine gesonderte Stechverbindungssicherung notwendig.
Kein mehrfacher Gebrauch der Stechverbindung möglich. Bestens geeignet um Übertragungsinfektionen auf allen Gebieten vorzubeugen.
Einfache Handhabung.
Einfach herzustellen und zu produzieren.

Weitere Einzelheiten der Erfindung sind der folgenden Beschreibung zu entnehmen, in der die Erfindung anhand der in der Zeichnung dargestellten Ausführungsbeispiele näher beschrieben und erläutert ist. Es zeigen:
- Figur 1: in einem Längsschnitt eine Entnahmevorrichtung in Form eines Verbindungsstücks für beispielsweise einen Infusions- oder Transfusionsbeutel,
- Figur 2: in einem Längsschnitt eine Entnahmevorrichtung in Form eines Verschlusses für beispielsweise eine Infusions- oder Transfusionsflasche,
- Figur 3: mehrere Ausführungsbeispiele eines bei einer Vorrichtung nach den Figuren 1 und 2 verwendeten axialen Sicherungsring in Draufsicht (rechte Spalte) und im Längsschnitt (linke Spalte),
- Figur 4: in einem Längsschnitt eine Zuspritzvorrichtung für beispielsweise einen Infusions- oder Transfusionsbeutel,
- Figur 5: in einem Längsschnitt eine Entnahmevorrichtung in Form eines Verbindungsstücks für beispielsweise einen Infusions- oder Transfusionsbeutel, ähnlich der Figur 1, jedoch gemäß einem anderen Ausführungsbeispiel, und
- Figur 6: in einem Längsschnitt eine Entnahmevorrichtung in Form eines Verschlusses für beispielsweise eine Infusions- oder Transfusionsflasche, ähnlich der Figur 2, jedoch gemäß einem anderen Ausführungsbeispiel.

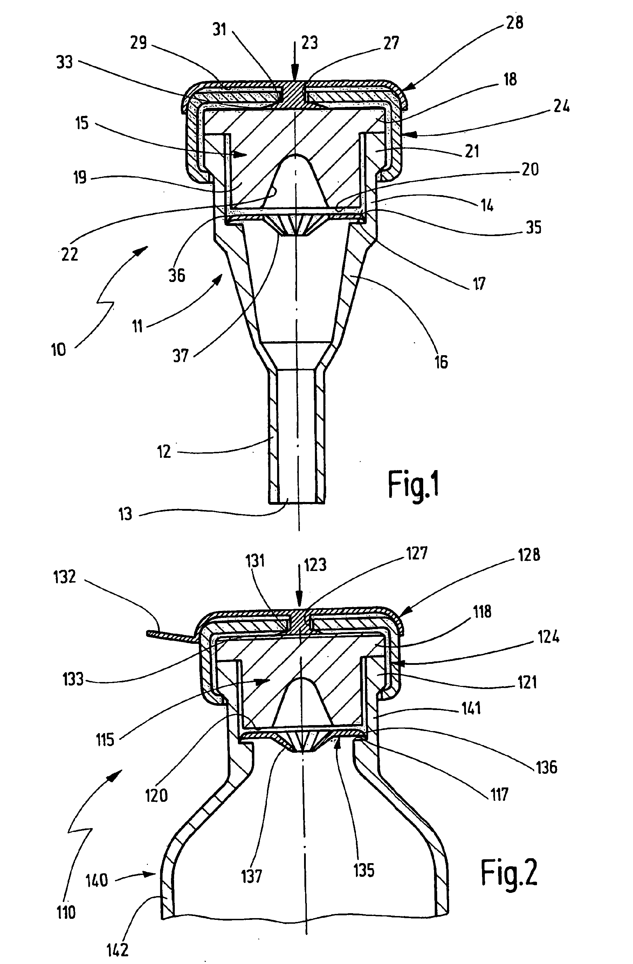

Die in Figur 1 dargestellte Vorrichtung in Form eines Verbindungsstücks 10 bzw. Konnektors kann mit einer beispielsweise eine medizinische Flüssigkeit enthaltenden und aus Kunststoff bestehenden, nicht dargestellten Verpackung, insbesondere mit einem Infusions- oder Transfusionsbeutel beispielsweise in der Weise fest verbunden werden, dass es mit seinem einen röhrchenförmigen Ende zwischen zwei Beutelwandteile so eingeschweißt oder eingeklebt wird, dass es mit dem Beutelinnenraum bzw. -inhalt in Verbindung steht. Das Verbindungsstück 10 kann direkt oder mittels einer kurzen flexiblen Zwischenleitung in das im Gebrauch untere Ende eines Beutels eingeschweißt oder verklebt werden. Derartige Vorrichtungen bzw. Verbindungsstücke 10 dienen dabei zum Entnehmen der im Beutel enthaltenden Flüssigkeit in der Weise, dass ein Spike bzw. Einstechdorn einer Tropfkammer eines nicht dargestellten Infusionsgerätes in das andere Ende des Verbindungsstücks 10 eingestochen wird. Einstechdorne sind an sich bekannt und beispielsweise in der DE 10 2006 053 219 A1 dargestellt und beschrieben.

Das Verbindungsstück 10 besitzt ein einstückiges hohles Anschlussteil 11 aus einem beispielsweise transparenten harten Kunststoff (PC, Acryl, usw.), das verpackungsseitig an seinem einen Ende 12 nach Art eines Röhrchens ausgebildet ist, das mit dem die Verpackung darstellenden betreffenden Beutel derart direkt oder mittelbar über eine flexible Leitung verbunden wird bzw. ist, dass die Mündung 13 des Röhrchens 12 mit dem Beutelinneren in Verbindung steht. Das der Mündung 13 abgewandte Ende 14 des Anschlussteils 11 ist zur Aufnahme einer Membran 15 ausgebildet. Zwischen dem durchmessergrößeren Aufnahmeteil 14 und dem durchmesserkleineren Röhrchen 12 ist ein Konusteil 16 vorgesehen, das mit seinem durchmessergrößeren Ende über eine Innenschulter 17 an das Aufnahmeteil 14 ansetzt und das mit seinem durchmesserkleineren anderen Ende in das Röhrchen 12 übergeht.

Die einstückige ein- oder mehrschichtige Membran 15 ist als Durchstechmembran ausgebildet und sitzt mit ihrem endseitigen radial abstehenden Ringrand 18 auf der Ringstirnfläche einer radialen Verdickung 21 des hohlen Aufnahmeteil 14 des Anschlussteils 11 auf. Die axiale Länge des in das Aufnahmeteil 14 ragenden Membranteils 19 ist geringer als der axiale Abstand zwischen Ringstirnfläche der Verdickung 21 des Aufnahmeteils 14 und der Innenschulter 17 zum Konusteil 16. Die beispielsweise Komposit-Membran 15 kann aus einem Kunststoff oder Kunststoffschichten unterschiedlicher Härte gebildet sein, indem ein radial innerer Mittenbereich durch einen weicheren Kunststoff bzw. Gummi als der radial äußere Bereich gebildet ist. Das Membranteil 19 besitzt von seinem dem Ringrand 18 abgewandten Ende ausgehend eine zur Längsachse symmetrische konische Ausnehmung 22. Das Durchstechen der Membran 15 mit einem Einstechdorn erfolgt in axialer Richtung gemäß Pfeil 23.

Die mit ihrem Ringrand 18 auf der Verdickung 21 des Anschlussteils 11 aufsitzende Membran 15 ist von einer Sicherungskappe 24 überdeckt, die außenumfangsseitig um und über sowie unter die Verdickung 21 des Aufnahmeteils 14 greift. Die Sicherungskappe 24 besitzt an ihrem die Membran 15 überdeckenden Bereich in axialer Mitte eine Kreisausnehmung 27, die die Fläche zum Durchstechen der Membran 15 mittels des nicht dargestellten Einstechdorns freilässt.

Die Kreisausnehmung 27 zur Durchführung des Einstechdorns oder der Einstechnadel (Spike) ist mit einem abnehmbaren, abziehbaren oder abknickbaren Originalitätsverschluss steril gesichert, der nach seiner Entfernung nicht wieder angebracht werden kann. Beim dargestellten Ausführungsbeispiel ist über der Sicherungskappe 24 eine insgesamt abnehmbare Originalitätskappe 28 angeordnet, die an ihrer Unterseite 29 mit kreisförmig angeordneten Widerhaken 31 versehen ist, die durch die Kreisausnehmung 27 und radial unter die Sicherungskappe 24 greifen. Die Originalitätskappe 28 kann beim dargestellten Ausführungsbeispiel durch einen an der Sicherungskappe 24 mittig angeordneten abdrehbaren oder abscherbaren Finger ersetzt sein, der die Kreisausnehmung 27 überdeckt und danach den Zugang zur Membran 15 freigibt.

Die durchstechbare Membran 15 ist auf ihrer in Einstechrichtung 23 oberen Fläche von einer Aluminiumfolie 33 überdeckt, die derart dünn ist, dass sie von einem Einstechdorn, einer Nadel oder dergleichen durchstechbar ist. Die Aluminiumfolie 33 ist entweder über die gesamte obere Fläche der Membran 15 oder lediglich über demjenigen Teil der oberen Fläche der Membran 15 vorgesehen, der den durchstechbaren Bereich der Membran 15 darstellt und der gegebenenfalls von der Kreisausnehmung 27 der Sicherungskappe 24 freigegeben ist. Die Aluminiumfolie 33 dient dazu, augenfällig bzw. sichtbar zu machen, wenn mit dem Infusions- bzw. Transfusionsbeutel bereits einmal ein Einstechdorn oder dergleichen verbunden gewesen ist, da dann die Aluminiumfolie 33 mit einer sichtbaren Perforierung bereits versehen ist.

Wie erwähnt, ergibt die Aluminiumfolie 33 eine zusätzliche Sicherheit gegen Manipulationen, da Einstiche mit einem Einstechdorn oder einer dünnen Nadel in die Einstichmembran 15 ohne eine derartige Aluminiumsicherheitsfolie 33 nicht sichtbar wäre, insbesondere dann, wenn das Membranmaterial sich entsprechend verschließt. Nur eine unversehrte Aluminiumsicherheitsfolie über der Einstichmembran gewährleistet einen Originalinfusionsbehälter bzw. -produkt.

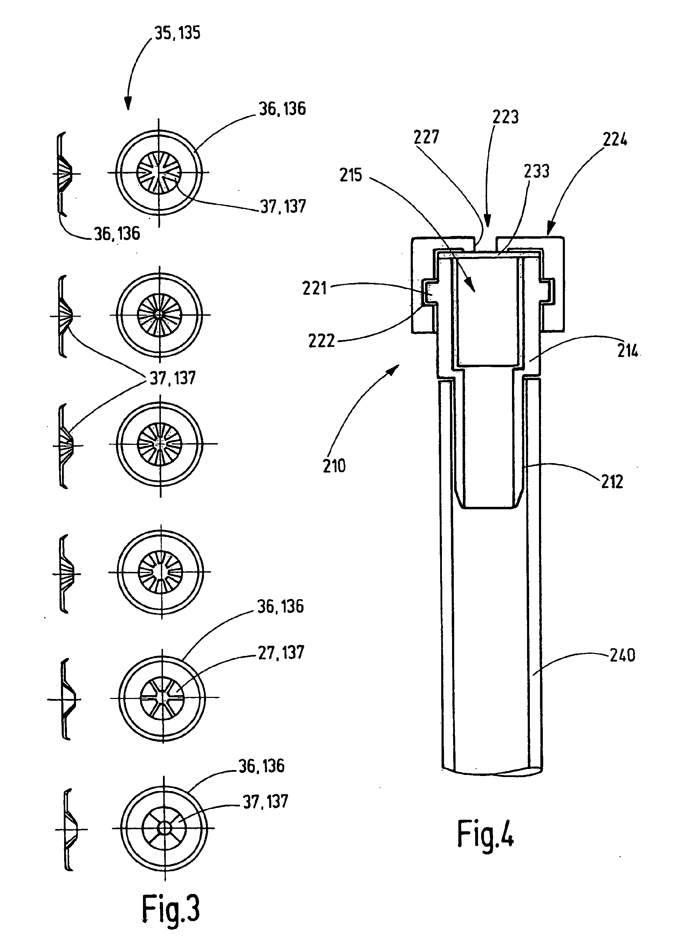

Zwischen der dem Ringrand 18 abgewandten Unterseite des Membranteils 19 und der Innenschulter 17 ist ein axial wirkendes Sicherungs- bzw. Verriegelungselement in Form eines Sicherungs- bzw. Verriegelungsring 35 angeordnet, dessen äußerer Ringrand 36 einerseits auf der Innenschulter 17 aufliegt und andererseits von der kreisringförmigen Stirn- bzw. Unterseite 20 des Membranteils 19 gehalten ist. Gemäß Figur 1 ist der aus rostfreiem Federstahl ausgebildete, axial wirkende Sicherungsring 35 geschlossen und mit radial nach innen gerichteten und über den Innenumfang des Ringrandes 36 gleichmäßig verteilt angeordneten Zähnen 37 oder Klammern versehen, die gegenüber dem ebenen Ringrand 36 in Einstechrichtung gemäß Pfeil 23 schräg radial nach innen und axial zum Konusteil 16 hin gerichtet sind. Die durch eine radiale Innenschlitzung eines Federstahlringes gebildeten Zähne 37 oder Klammern besitzen konkav geformte freie innere Enden die in Umfangsrichtung einen durch die Innenschlitzung unterbrochenen Kreis bilden. In Figur 3 sind Ausführungsbeispiele hierzu dargestellt. Beispielsweise sind die Zähne 37 in Draufsicht auch statt trapezförmig zu ihrem inneren Ende hin dreieckförmig und damit spitz bzw. nach innen konisch zulaufend ausgebildet. Vorzugsweise ist eine Vielzahl von Zähnen 37 vorgesehen, wobei die Mindestzahl zwei ist. Der mit Zähnen 37 versehene Sicherungsring kann auch an einem Umfangsbereich offen sein, so dass er dort radial federnd auseinander drängbar ist.

Die in Figur 2 gemäß einem anderen Ausführungsbeispiel dargestellte Vorrichtung ist in Form eines Verschlusses 110 einer Infusions- oder Transfusionsflasche 140 ausgeführt, die die vorzugsweise Einwegverpackung für insbesondere medizinische Flüssigkeiten bildet. Der Verschluss 110 dient wie das Verbindungsstück 10 gemäß dem ersten Ausführungsbeispiel zum Entnehmen der in der Flasche 110 enthaltenen Flüssigkeit, wobei auch hier zum Entnehmen der in der Flasche enthaltenen Flüssigkeit in den Verschluss 110 ein Spike bzw. Einstechdorn einer Tropfkammer eines nicht dargestellten Infusionsgerätes eingestochen wird.

Der Verschluss 110 ist in und auf den gegenüber dem Flaschenkorpus 142 verjüngten Flaschenhals 141 der Flasche 140 eingesetzt. Der Flaschenhals 141 ist ähnlich dem Aufnahmeteil 14 des Anschlussteils 11 des ersten Ausführungsbeispiels ausgebildet, so dass hinsichtlich der Halterung des mit einer durchstechbaren Membran 115 versehenen Verschlusses 110 im und am Flaschenhals 141 im Wesentlichen hierauf verwiesen werden kann. Die Membran 115 besitzt ebenfalls einen Ringrand 118, mit dem die Membran 115 auf der Stirnfläche des Ringrandes 121 des Flaschenhalses 141 aufsitzt. Der Außenumfang der kreisringförmigen Stirnfläche des Flaschenhalses 141 und des Ringrands 118 der Membran 115 sind im Wesentlichen gleich. Die Membran 115 ist bezüglich Form und Material wie die Membran 15 des ersten Ausführungsbeispiels ausgebildet. Die Oberseite der Membran 115 ist wie beim ersten Ausführungsbeispiel mit einer Aluminiumsicherheitsfolie 133 ganz oder teilweise überzogen. Mittels einer Sicherungskappe 124, die beim Ausführungsbeispiel den Außenumfang des außen hinterschnittenen Flaschenhalses 141 umfassend herumgeht, ist die Membran 115 auf und im Flaschenhals 141 fixiert. Die Sicherungskappe 124 ist wie beim ersten Ausführungsbeispiel von einer Originalitätskappe 128 überdeckt, die durch eine Kreisausnehmung 127 in der Sicherungskappe 124 mit einem einstückigen Ansatz geführt und zwischen der Unterseite der Sicherungskappe 124 und der Oberseite der Aluminiumfolie 133 mittels Widerhaken 131 fixiert ist. Beim dargestellten Ausführungsbeispiel besitzt die Originalitätskappe 128 eine an einem Umfang abstehende Lasche 132, mittels der die Originalitätskappe 128 abgenommen werden kann.

Der Flaschenhals 141 ist vor seinem Übergang zum Flaschenkorpus 142 mit einer inneren Schulter 117 versehen, auf der, wie beim ersten Ausführungsbeispiel der Figur 1 der Ringrand 136 eines der Verriegelung- bzw. Sicherungsring 135 aufsitzt der zwischen innerer Schulter 117 und Unterseite der Membran 115 gehalten ist. Der mit radialen Zähnen 137 versehene Sicherungsring 135 ist entsprechend dem Sicherungsring 35 des ersten Ausführungsbeispiels ausgebildet und kann gemäß Figur 3 verschiedene Formen annehmen. Die Wirkung dieses Sicherungsrings 135 ist dieselbe wie beim ersten Ausführungsbeispiel der Figur 1.

Die Ausgestaltung des Originalitätsverschlusses 128, kann, wie zum ersten Ausführungsbeispiel beschrieben, in unterschiedlicher Weise gelöst werden, beispielsweise durch einen abbrechbaren Zapfen, der an der Sicherungskappe 124 vorgesehen ist und damit die Originalitätskappe 128 ersetzt.

Wird somit ein üblicherweise axial leicht konisch zulaufender, aus Kunststoff bestehender Einstechdorn verwendet und gemäß Pfeil 23 bzw. 123 durch die Membran 15 bzw. 115 hindurch gestochen, gelangt der in der Membran 15 bzw. 115 dicht umschlossene Einstechdorn zwischen die Zähne 37 bzw. 137 des Verriegelungs- bzw. Sicherungsringes 35 bzw. 135, wobei deren Schneiden sich in den Außenumfang des Einstechdorns leicht eingraben. Auf diese Weise erfolgt nicht nur eine reibschlüssige Verbindung sondern auch eine etwa formschlüssige Verbindung zwischen Sicherungsring 35 bzw. 135 und Einstechdorn, die einerseits verhindert, dass der Einstechdorn axial wieder zurückgezogen werden kann, weil sich die Zähne 37 bzw. 137 entgegen ihrer Neigungsrichtung weiter in die Außenwandung des Einstechdorns eingraben würden, und andererseits ein Verdrehen des Einstechdorns während und nach dem Einstechen verhindert. Der Einsteckdorn wird im Ring 35 bzw. 135 axial und in Umfangsrichtung festgehalten.

Beim dargestellten Ausführungsbeispiel ist das hohle Anschlussteil 11 des Verbindungsstücks 10 vorzugsweise aus Polyethylen, Polypropylen, Polycarbonat (PC) oder PVC. Der Sicherungs- bzw. Verriegelungsring 35 bzw. 135 ist aus nicht rostendem Federstahl medizinischer Qualität. Die Sicherungskappe 24 bzw. 124 ist aus Polypropylen oder PVC, wenn sie angeschweißt oder angeklebt wird, bzw. aus Aluminium oder nicht rostendem Stahl, wenn sie aufgepresst wird. Die Originalitätskappe 28 bzw. 128 ist aus PVC oder Polyethylen oder Polypropylen oder dergleichen hergestellt. Die Membran 15 bzw. 115 ist aus Latex oder einem Chromobutylgummi oder einem ähnlichen in der Medizin verwendeten Werkstoff. Alle Bauteile müssen unter Wärme oder Dampf bei mindestens 121°C oder durch Gas oder Hochfrequenz sterilisierbar sein.

Insbesondere bei Infusions- oder Transfusionsbeuteln ist es üblich, außer der Entnahmeöffnung, die mit dem Verbindungsstück 10 gemäß Figur 1 verbunden ist, eine weitere Öffnung vorzusehen, die dem Zuspritzen eines Medikamentes dient und die beispielsweise mit einer flexiblen Leitung 249 flüssigkeitsdicht verbunden ist. In diese flexible Leitung 249 ist eine Zuspritzvorrichtung eingesetzt, die als Verbindungsstück 210 ausgebildet ist. Das Verbindungsstück 210 besitzt ein Rohrteil 212, mit dem es in die flexible Leitung 249 flüssigkeitsdicht einsetzbar ist, und ein Aufnahmeteil 214, dessen Außendurchmesser etwas größer ist als der des Rohrteils 212. Das Aufnahmeteil 214 besitzt einen außenumfangsseitigen Ringansatz 221, über den eine mit einer Ringausnehmung 222 versehene Sicherungskappe 224 gebracht und gegen Abnehmen gesichert ist. Die Sicherungskappe 224 kann somit gegenüber dem Verbindungsstück 210 verdreht, jedoch nicht axial abgenommen werden.

In das Aufnahmeteil 214 ist eine durchstechbare Membran 215 eingesetzt, die zylindrisch ausgebildet ist, die jedoch auch in der in den Figuren 1 oder 2 dargestellten Weise mit einem Ringrand versehen sein kann. Die Membran 215 ist ebenfalls, wie erwähnt, als Durchstechmembran ausgebildet, die beispielsweise von der Nadel einer Spritze durchstochen werden kann, um ein Medikament in die im Infusions- oder Transfustionsbeutel enthaltene medizinische Flüssigkeit einspritzen zu können. Zum Sichtbarmachen der Originalität ist die Oberseite der Membran 215 ganz oder teilweise (nur im Bereich der Kreisausnehmung 227 der Kappe 224) ebenfalls von einer durchstechbar dünnen Aluminiumfolie 233 überzogen. Das Verbindungsstück 210 besitzt keinen Sicherungsring, da die Nadel der Spritze nach erfolgtem Zuspritzen wieder herausgezogen werden muss.

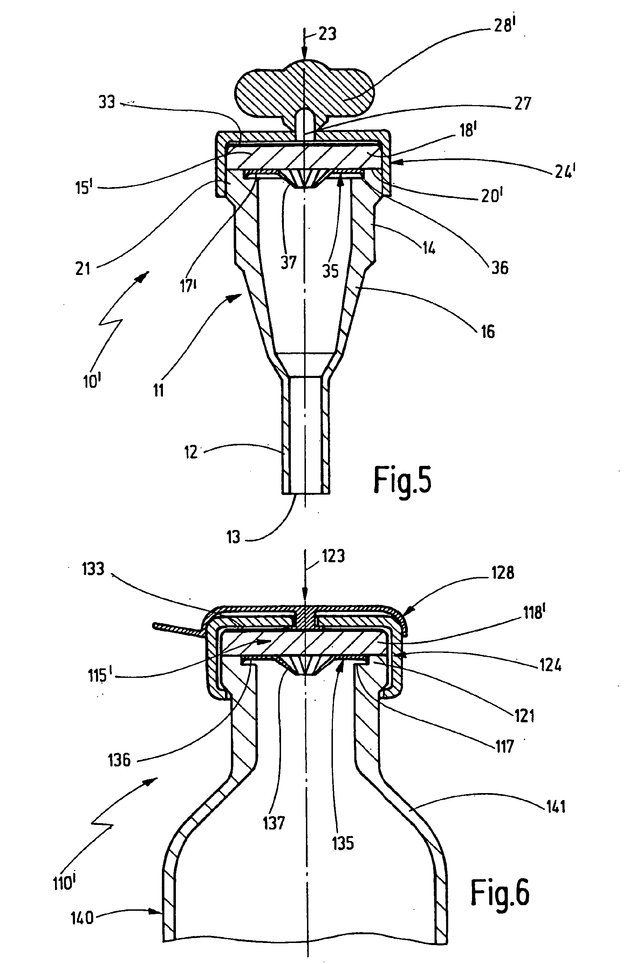

Die in Figur 5 dargestellte Vorrichtung in Form eines Verbindungsstücks 10' bzw. Konnektors kann wie das Verbindungsstück 10 nach Figur 1 mit einer beispielsweise eine medizinische Flüssigkeit enthaltenden und aus Kunststoff bestehenden, nicht dargestellten Verpackung, insbesondere mit einem Infusions- oder Transfusionsbeutel beispielsweise in der Weise fest verbunden werden, dass es mit seinem einen röhrchenförmigen Ende zwischen zwei Beutelwandteile so eingeschweißt oder eingeklebt wird, dass es mit dem Beutelinnenraum bzw. -inhalt in Verbindung steht. Das Verbindungsstück 10 kann direkt oder mittels einer kurzen flexiblen Zwischenleitung in das im Gebrauch untere Ende eines Beutels eingeschweißt oder verklebt werden. Derartige Vorrichtungen bzw. Verbindungsstücke 10' dienen dabei zum Entnehmen der im Beutel enthaltenden Flüssigkeit in der Weise, dass ein Spike bzw. Einstechdorn einer Tropfkammer eines nicht dargestellten Infusionsgerätes in das andere Ende des Verbindungsstücks 10' eingestochen wird.

Das Verbindungsstück 10' besitzt ebenfalls ein einstückiges hohles Anschlussteil 11 aus einem beispielsweise transparenten harten Kunststoff (PC, Acryl, usw.), das verpackungsseitig an seinem einen Ende 12 nach Art eines Röhrchens ausgebildet ist, das mit dem die Verpackung darstellenden betreffenden Beutel derart direkt oder mittelbar über eine flexible Leitung verbunden wird bzw. ist, dass die Mündung 13 des Röhrchens 12 mit dem Beutelinneren in Verbindung steht. Das der Mündung 13 abgewandte zylindrische Teil 14 des Anschlussteils 11 geht über ein Konusteil 16 in das durchmesserkleinere Röhrchen 12 über.

Eine einstückige ein- oder mehrschichtige Membran 15' besitzt die Form einer scheibenartigen Flachmembran und ist als Durchstechmembran ausgebildet. Sie sitzt mit der Unterseite 20' eines Ringrandbereichs 18' auf der Ringstirnfläche einer radialen Verdickung 21 des offenen Endes des hohlen Aufnahmeteil 14 des Anschlussteils 11 auf. Die Verdickung 21 ist radial innen mit einer eine axiale Innenschulter 17' bildenden Ringaussparung versehen. Die beispielsweise Komposit-Flachmembran 15' kann aus einem Kunststoff oder Kunststoffschichten unterschiedlicher Härte gebildet sein, indem ein radial innerer Mittenbereich durch einen weicheren Kunststoff bzw. Gummi als der radial äußere Bereich gebildet ist. Das Durchstechen der Flachmembran 15' mit einem Einstechdorn erfolgt in axialer Richtung gemäß Pfeil 23.

Die mit ihrem Ringrand 18' auf der Ringstirnfläche der Verdickung 21 des Anschlussteils 11 aufsitzende Flachmembran 15' ist von einer Sicherungskappe 24' überdeckt, die außenumfangsseitig um und über sowie unter die Verdickung 21 des Aufnahmeteils 14 greift und dort beispielsweise verklebt ist. Die Sicherungskappe 24' besitzt an ihrem die Membran 15' überdeckenden Bereich in axialer Mitte eine Kreisausnehmung 27, die die Fläche zum Durchstechen der Flachmembran 15' mittels des nicht dargestellten Einstechdorns freilässt. Die Kreisausnehmung 27 zur Durchführung des Einstechdorns oder der Einstechnadel (Spike) ist mit einem abnehmbaren, abziehbaren oder abknickbaren Originalitätsverschluss steril gesichert, der nach seiner Entfernung nicht wieder angebracht werden kann. Beim dargestellten Ausführungsbeispiel ist mit der Sicherungskappe 24' eine flügelartiges Originalitätselement 28' als Originalitätsverschluss verbunden, das wenn es um die Achse der Kreisausnehmung 27 gedreht wird, abreißt und die Kreisausnehmung 27 unwiederbringlich freilegt. Das Originalitätselement 28' kann stattdessen durch einen an der Sicherungskappe 24' mittig angeordneten abknickbaren bzw. abscherbaren Finger eingesetzt sein, der die Kreisausnehmung 27 überdeckt und danach über die Kreisausnehmung 27 den Zugang zur Flachmembran 15' freigibt.

Die durchstechbare Flachmembran 15' ist auch hier auf ihrer in Einstechrichtung 23 oberen Fläche von einer Aluminiumfolie 33 überdeckt, die derart dünn ist, dass sie von einem Einstechdorn, einer Nadel oder dergleichen durchstechbar ist, wodurch augenfällig bzw. sichtbar gemacht wird, wenn mit dem Infusions- bzw. Transfusionsbeutel bereits einmal ein Einstechdorn oder dergleichen verbunden gewesen ist, da dann die Aluminiumfolie 33 mit einer sichtbaren Perforierung unumkehrbar versehen ist.

Gemäß Figur 5 ist unterhalb der Flachmembran 15' in der die Innenschulter 17' beinhaltenden Ringaussparung der axial wirkende Sicherungs- und Verriegelungsring 35 axial und radial fixiert angeordnet, dessen äußerer Ringrand 36 einerseits auf der Innenschulter 17' aufliegt und andererseits von der Unterseite 20' der Flachmembran 15' gehalten ist. Gemäß Figur 5 ist der aus rostfreiem Federstahl oder einem anderen geeigneten Material hergestellte, axial wirkende Sicherungsring 35 gemäß Figur 3 mit radial nach innen gerichteten und über den Innenumfang des Ringrandes 36 gleichmäßig verteilt angeordneten Zähnen 37 oder Klammern versehen.

Die in Figur 6 gemäß einem weiteren Ausführungsbeispiel dargestellte Vorrichtung ist ähnlich der Figur 2 in Form eines Verschlusses 110' einer Infusions- oder Transfusionsflasche 140 ausgeführt, die die vorzugsweise Einwegverpackung für insbesondere medizinische Flüssigkeiten bildet. Der Verschluss 110' dient wie der Verschluss 110 gemäß dem zweiten Ausführungsbeispiel der Figur 2 zum Entnehmen der in der Flasche 140 enthaltenen Flüssigkeit, wobei auch hier zum Entnehmen der in der Flasche enthaltenen Flüssigkeit in den Verschluss 110 ein Spike bzw. Einstechdorn einer Tropfkammer eines nicht dargestellten Infusionsgerätes eingestochen wird.

Wie beim Ausführungsbeispiel nach Figur 5 besitzt der Verschluss 110' eine durchstechbare Flachmembran 115', die mit einem Ringrandbereich 118' auf der Stirnfläche des radial verdickten Ringrandes 121 des Flaschenhalses 141 unmittelbar aufsitzt. Wie beim vorhergehenden Ausführungsbeispiel ist die kreisringförmige Stirnfläche des Flaschenhalses 141 radial innen mit einer Ringaussparung versehen, auf deren axialer Innenschulter 117' unter der Flachmembran 115' der Sicherungsring 135 angeordnet ist, dessen Ringrand 136 und Zähne 137 entsprechend Figur 3 gestaltet sind.

Die Flachmembran 115' ist bezüglich Form und Material wie die Flachmembran 15' des vorstehenden Ausführungsbeispiels nach Figur 1 ausgebildet. Die Oberseite der Membran 115' ist wie bei den Ausführungsbeispielen der Figuren 1 und 2 und dem nach Figur 5 unmittelbar mit einer Aluminiumsicherungsfolie 133 ganz oder teilweise überzogen. Die hier vorgesehene Sicherungskappe 124 entspricht der beim Ausführungsbeispiel nach Figur 2 vorgesehenen; sie kann jedoch auch dem Ausführungsbeispiel der Figur 5 entsprechen. Entsprechendes gilt für das an der Sicherungskappe 124 vorgesehene Originalitätselement 128.

Wird somit ein üblicherweise axial leicht konisch zulaufender, aus Kunststoff bestehender Einstechdorn verwendet und gemäß Pfeil 23 bzw. 123 durch die Membran 15' bzw. 115' und ggf. zuerst durch die Aluminiumfolie 33 bzw. 133 hindurch gestochen, gelangt der in der Flachmembran dicht umschlossene Einstechdorn zwischen die Zähne 37 bzw. 137 des Verriegelungs- bzw. Sicherungsringes 35' bzw. 135', wobei deren Schneiden sich in den Außenumfang des Einstechdorns, Spike oder dergleichen, leicht eingraben. Auf diese Weise erfolgt nicht nur eine reibschlüssige Verbindung sondern auch eine etwa formschlüssige Verbindung zwischen Sicherungsring 35 bzw. 135 und Einstechdorn, Spike oder dergleichen die einerseits verhindert, dass der Einstechdorn oder dergleichen axial wieder zurückgezogen werden kann, weil sich die Zähne 37 bzw. 137 entgegen ihrer Neigungsrichtung weiter in die Außenwandung des Einstechdorns oder dergleichen eingraben würden, und andererseits ein Verdrehen des Einstechdorns oder dergleichen während des und nach dem Einstechen verhindert.

Das den Einsteckdorn unumkehrbar festhaltende Sicherungs- bzw. Verriegelungselement kann statt als geschlossener oder offener Ring auch U-förmig mit nach innen weisenden Zähnen oder als Reif mit inneren gegeneinander konisch einwärts gerichteten Schneiden ausgebildet sein.

## Patentansprüche

1. Für die Entnahme von insbesondere medizinischen Flüssigkeiten aus vorzugsweise Einwegverpackungen, wie Infusions- oder Transfusionsbeutel oder -flaschen, geeignete Vorrichtung entweder in Form eines Verbindungsstücks (10) mit einem hohlen Anschlussteil (11), der eine verpackungsseitige Mündung (13) und eine anschlussseitige Aufnahme mit einer durchstechbaren Membran (15) aufweist, oder in Form eines Verschlusses (110) mit einer durchstechbaren Membran (115) im Flaschenhals (141), wobei die jeweilige Membran (15, 115) zur abdichtend umschließenden Aufnahme eines Durchstechdorn oder dergleichen zur Entnahme der Flüssigkeit dient, **dadurch gekennzeichnet, dass** in Einstechrichtung (23, 123) hinter der Membran (15, 115) und vor der verpackungsseitigen Mündung (13) bzw. vor dem Flaschenkorpus (142) in dem hohlen Anschlussteil (11) bzw. dem Flaschenhals (141) ein axiales Sicherungs- bzw. Verriegelungselement (35, 135) zur axial unumkehrbaren Aufnahme des Einstechdorns oder dergleichen ortsfest gehalten ist.

2. Vorrichtung nach Anspruch 1, **dadurch gekennzeichnet, dass** die durchstechbare Membran als Flachmembran (15', 115') ausgebildet ist.

3. Vorrichtung nach Anspruch 1 oder 2, **dadurch gekennzeichnet, dass** das aus nicht rostendem Federstahl gebildete Sicherungs- bzw. Verriegelungselement (35, 135) als offene oder geschlossene Ringscheibe mit über den Innenumfang verteilt angeordneten, radial nach innen vorstehenden Zähnen (37, 137) oder Klammern ausgebildet ist.

4. Vorrichtung nach Anspruch 3, **dadurch gekennzeichnet, dass** die Zähne (37, 137) oder dergleichen im Umfangsabstand gleichmäßig verteilt angeordnet sind.

5. Vorrichtung nach Anspruch 2 oder 3, **dadurch gekennzeichnet, dass** die Zähne (37, 137) oder dergleichen mit ihren radial nach innen weisenden freien Enden einen Kreis bilden und/oder zu ihrem radial inneren Ende hin spitz zulaufen.

6. Vorrichtung nach mindestens einem der Ansprüche 2 bis 5, **dadurch gekennzeichnet, dass** die Zähne (37, 137) oder dergleichen in Einstechrichtung (23, 123) vorzugsweise konisch geneigt verlaufen.

7. Vorrichtung nach mindestens einem der Ansprüche 2 bis 6, **dadurch gekennzeichnet, dass** die Zähne (37, 137) oder dergleichen sowohl ein Zurückziehen als auch ein Verdrehen des Einstechdorns oder dergleichen verhindern.

8. Vorrichtung nach mindestens einem der vorhergehenden Ansprüche, **dadurch gekennzeichnet, dass** im hohlen Anschlussteil (11) bzw. im Flaschenhals (141) zwischen einem etwa zylindrischen Aufnahmeraum (14, 114) für die Membran (15, 115) und einem daran anschließenden konischen Durchgangsraum (16) zur verpackungsseitigen Mündung (13) bzw. dem Flaschenkorpus (142) oder unmittelbar unterhalb der ringförmigen Stirnfläche für die Auflage der Flachmembran (15', 115') eine Innenschulter (17, 117 bzw. 17', 117') vorgesehen ist, auf der sich das Sicherungs- bzw. Verriegelungselement (35, 135) axial abstützt.

9. Vorrichtung nach Anspruch 8, **dadurch gekennzeichnet, dass** das Sicherungs- bzw. Verriegelungselement (35, 135) zwischen der Innenschulter (17, 117) und dem zugewandten Ende der Membran (15, 15', 115, 115') eingespannt ist.

10. Vorrichtung nach mindestens einem der vorhergehenden Ansprüche, **dadurch gekennzeichnet, dass** die durchstechbare Membran(15, 15', 115, 115') zumindest im zugängigen Durchstechbereich von einer durchstechbaren dünnen Aluminiumfolie (33, 133) überzogen ist.

11. Vorrichtung nach Anspruch 10, **dadurch gekennzeichnet, dass** die Aluminiumfolie (33, 133) mit der Oberfläche der Membran (15, 15', 115, 115') verklebt oder verschweißt ist.

12. Verpackung für insbesondere medizinische Flüssigkeiten in Form eines vorzugsweise Einweg-Infusions- oder Transfusionsbeutels mit einer Vorrichtung in Form eines Verbindungsstücks (10, 10') nach Anspruch 1 und/oder mindestens einem der folgenden Ansprüche 2 bis 11.

13. Verpackung in Form eines Einweg-Infusions- oder Transfusionsbeutels, insbesondere nach Anspruch 12, mit einer zum Zuspritzen eines Medikamentes geeigneten Vorrichtung, die eine durchstechbare Membran (215) zur abdichtenden umschließenden Aufnahme einer Injektionsnadel einer Spritze oder dergleichen aufweist, **dadurch gekennzeichnet, dass** die durchstechbare Membran (215) zumindest im zugängigen Durchstechbereich von einer durchstechbar dünnen Aluminiumfolie (233) überzogen ist.

14. Verpackung nach Anspruch 13, **dadurch gekennzeichnet, dass** die Aluminiumfolie (233) mit der Oberfläche der Membran (15, 115) verklebt oder verschweißt ist.

15. Verpackung für insbesondere medizinische Flüssigkeiten in Form einer Infusions- oder Transfusionsflasche (140) mit einer Vorrichtung in Form eines Verschlusses (110, 110') nach Anspruch 1 und/oder mindestens einem der folgenden Ansprüche 2 bis 11.

## Claims

1. A device that is suitable for the removal of medical fluids from disposable packaging, such as infusion or transfusion bags or bottles, either in the form of a connecting piece (10) having a hollow connecting part (11), which has an aperture (13) on the packaging side and a receptacle on the connecting side having a pierceable membrane (15), or in the form of a closure (110) having a pierceable membrane (115) within the neck of the bottle (141), whereby both of the membranes (15, 115) receive a spike or the like in a sealingly enclosed manner in order to remove the fluid, wherein in the puncture direction (23, 123) downstream of the membrane (15, 115) and upstream of the aperture (13) on the packaging side, or upstream of the body of the bottle (142) within the hollow connecting part (11) or within the neck of the bottle (141) an axial retaining and locking element (35, 135) is fixedly supported to facilitate the axially non-reversible reception of the spike or the like.

2. The device as recited in Claim 1, wherein the pierceable membrane is configured as a flat membrane (15', 115').

3. The device as recited in Claim 1 or 2, wherein the retaining and locking element (35, 135), which is made of stainless spring steel, is configured as an open or closed ring disk having teeth (37, 137) or clamps, which protrude radially to the inside and are distributed over the interior circumference.

4. The device as recited in Claim 3, wherein the teeth (37, 137) or the like are distributed uniformly over the circumference.

5. The device as recited in Claim 2 or 3, wherein the teeth (37, 137) or the like form a circle with their free ends, which point radially towards the interior and/or taper to a point at their radially interior end.

6. The device as recited in any of Claims 2 to 5, wherein the teeth (37, 137) or the like in the puncture direction (23, 123) have a conical, slanted shape.

7. The device as recited in any of Claims 2 to 6, wherein the teeth (37, 137) or the like prevent both the removal as well as the rotation of the spike or the like.

8. The device as recited in any of the preceding claims, wherein within the hollow connecting part (11) or within the neck of the bottle (141) between an approximately cylindrical receiving chamber (14, 114) for the membrane (15, 115) and a conical transition chamber (16) connected thereto leading to the aperture (13) on the packaging side or the body of the bottle (142) or immediately beneath the angular end face for the placement of the flat membrane (15', 115') an internal shoulder (17, 117 or 17', 117') is provided, on which the retaining and locking element (35, 135) is axially supported.

9. The device as recited in Claim 8, wherein the retaining and locking element (35, 135) is clamped between the internal shoulder (17, 117) and the facing end of the membrane (15, 15', 115, 115').

10. The device as recited in any of the preceding claims, wherein the pierceable membrane (15, 15', 115, 115') is covered by a pierceable, thin aluminum foil (33, 133) at least in the area accessible to puncture.

11. The device as recited in Claim 10, wherein the aluminum foil (33, 133) is glued or welded to the surface of the membrane (15, 15', 115, 115').

12. A packaging for medical fluids in the form of a disposable infusion or transfusion bag having a device in the form of a connecting piece (10, 10') as recited in Claim 1 and/or any of subsequent Claims 2 to 11.

13. The packaging in the form of a disposable infusion or transfusion bag as recited in Claim 12, having a device that is suited for the injection of a medicine and that has a pierceable membrane (215) for the sealingly enclosed reception of a hypodermic needle containing an injection or the like, wherein the pierceable membrane (215) is covered by a pierceable, thin aluminum foil (233) at least in the area accessible to puncture.

14. The packaging as recited in Claim 13, wherein the aluminum foil (233) is glued or welded to the surface of the membrane (15, 115).

15. The packaging for medical fluids in the form of an infusion or transfusion bottle (140) having a device in the form of a closure (110, 110') as recited in Claim 1 and/or any of subsequent Claims 2 to 11.

## Revendications

1. Dispositif approprié pour le prélèvement de fluides, en particulier médicaux, contenus dans des emballages de préférence à usage unique, tels que poches ou bouteilles de perfusion ou de transfusion, et se présentant soit sous forme d'une pièce de jonction (10) ayant une partie creuse de raccordement (11) qui présente une bouche (13) côté emballage et un logement côté raccord avec une membrane (15) apte à être percée, soit sous forme d'une fermeture (110) avec une membrane (115) apte à être percée, à l'intérieur du goulot de bouteille (141), ladite membrane (15, 115) respective servant à recevoir une pointe de perforation ou similaire pour le prélèvement du fluide, de manière à l'enfermer avec étanchéité, **caractérisé par le fait que**, dans la direction de perforation (23, 123) en aval de ladite membrane (15, 115) et en amont de la bouche (13) côté emballage ou bien en amont du corps de bouteille (142), un élément de sécurité ou bien de verrouillage axial (35, 135) destiné à recevoir d'une manière axialement irréversible ladite pointe de perforation ou similaire est tenu à poste fixe dans ladite partie creuse de raccordement (11) ou bien dans ledit goulot de bouteille (141).

2. Dispositif selon la revendication 1, **caractérisé par le fait que** ladite membrane apte à être percée est réalisée en tant que membrane plate (15', 115').

3. Dispositif selon la revendication 1 ou 2, **caractérisé par le fait que** ledit élément de sécurité ou bien de verrouillage (35, 135) fait d'un acier à ressorts inoxydable est réalisé en tant que disque annulaire ouvert ou fermé ayant des dents (37, 137) ou pinces réparties sur la circonférence intérieure et se projetant radialement vers l'intérieur.

4. Dispositif selon la revendication 3, **caractérisé par le fait que** les dents (37, 137) ou similaires sont disposées, quant à la distance circonférentielle, de manière à être réparties uniformément.

5. Dispositif selon la revendication 2 ou 3, **caractérisé par le fait que** lesdites dents (37, 137) ou similaires forment un cercle avec leurs extrémités libres montrant radialement vers l'intérieur et/ou s'effilent vers leur extrémité radialement intérieure.

6. Dispositif selon l'une au moins des revendications 2 à 5, **caractérisé par le fait que** lesdites dents (37, 137) ou similaires s'étendent dans ladite direction de perforation (23, 123) de préférence à inclinaison conique.

7. Dispositif selon l'une au moins des revendications 2 à 6, **caractérisé par le fait que** lesdites dents (37, 137) ou similaires empêchent aussi bien un retrait qu'une rotation de ladite pointe de perforation ou similaire.

8. Dispositif selon l'une au moins des revendications précédentes, **caractérisé par le fait que** dans ladite partie creuse de raccordement (11) ou bien à l'intérieur du goulot de bouteille (141), entre un espace de réception à peu près cylindrique (14, 114) pour ladite membrane (15, 115) et un espace conique contigu de passage (16) à ladite bouche (13) côté emballage ou bien ledit corps de bouteille (142) ou immédiatement au-dessous de la surface frontale annulaire pour l'appui de la membrane plate (15', 115'), est prévue une épaule intérieure (17, 117 ou bien 17', 117') sur laquelle ledit élément de sécurité ou bien de verrouillage (35, 135) s'appuie axialement.

9. Dispositif selon la revendication 8, **caractérisé par le fait que** ledit élément de sécurité ou bien de verrouillage (35, 135) est serré entre ladite épaule intérieure (17, 117) et l'extrémité de la membrane (15, 15', 115, 115'), qui est tournée vers lui.

10. Dispositif selon l'une au moins des revendications précédentes, **caractérisé par le fait que** la membrane (15, 15', 115, 115') apte à être percée est revêtue, au moins dans la zone de perforation accessible, d'une mince feuille en aluminium (33, 133) apte à être percée.

11. Dispositif selon la revendication 10, **caractérisé par le fait que** ladite feuille en aluminium (33, 133) est collée ou soudée avec la surface de ladite membrane (15, 15', 115, 115').

12. Emballage pour fluides en particulier médicaux, sous forme d'une poche de perfusion ou de transfusion de préférence à usage unique, comprenant un dispositif sous forme d'une pièce de jonction (10, 10') selon la revendication 1 et/ou l'une au moins des revendications suivantes 2 à 11.

13. Emballage sous forme d'une poche de perfusion ou de transfusion à usage unique, en particulier selon la revendication 12, comprenant un dispositif apte à injecter un médicament qui présente une membrane (215) apte à être percée et servant à recevoir une aiguille d'injection d'une seringue ou similaire de manière à l'enfermer avec étanchéité, **caractérisé par le fait que** ladite membrane (215) apte à être percée est revêtue, au moins dans la zone de perforation accessible, d'une mince feuille en aluminium (233) apte à être percée.

14. Emballage selon la revendication 13, **caractérisé par le fait que** ladite feuille en aluminium (233) est collée ou soudée avec la surface de ladite membrane (15, 115).

15. Emballage pour fluides en particulier médicaux, sous forme d'une bouteille de perfusion ou de transfusion (140), comprenant un dispositif sous forme d'une fermeture (110, 110') selon la revendication 1 et/ou l'une au moins des revendications suivantes 2 à 11.
